# FASCICULE DE BREVET EUROPEEN

(11) **EP 0 624 361 B1**
(45) Date de publication et mention de la délivrance du brevet: **17.09.1997**
(21) Numéro de dépôt: 94400971.1
(22) Date de dépôt: 04.05.1994
(51) Int. Cl.: A61K 7/021, A61K 7/032, A61K 7/035

(54) **Procédé de fabrication d'une composition cosmétique solide à l'aide de plâtre et composition cosmétique ainsi obtenue**
Herstellungsverfahren einer festen kosmetischen Zusammensetzung mit Hilfe von Gips und so hergestellte kosmetische Zusammenstellung
Method of manufacturing a solid cosmetic composition with the help of plaster and the obtained cosmetic composition

(30) Priorité: 13.05.1993 FR 9305774
(43) Date de publication de la demande: 17.11.1994
(73) Titulaire: L'OREAL, 75008 Paris (FR)
(72) Inventeur: Lecomte, Sophie, F-75013 Paris (FR); Le Gars, Gwenola, F-75013 Paris (FR)
(74) Mandataire: Peuscet, Jacques

(56) Documents cités:
- EP-A- 0 095 889
- WO-A-86/00798
- US-A- 4 724 138
- US-A- 5 049 376

## Description

La présente invention concerne un procédé de fabrication d'une composition cosmétique solide à l'aide de plâtre et une composition cosmétique obtenue par ce procédé.

Il est connu de fabriquer des compositions cosmétiques sous forme solide à l'aide de plâtre. Ces compositions cosmétiques peuvent être notamment des fards à paupières, des fards à joues, des poudres pour le visage ou pour le corps ; elles peuvent être sous forme de sticks, de crayons ou de pains. L'utilisation du plâtre pour la fabrication de ces compositions solides est, par exemple, décrite dans EP-A 0 036 698. Selon les procédés connus, une phase contenant du plâtre est mélangée avec une phase aqueuse pour obtenir une pâte que l'on met en forme par moulage, la réaction d'hydratation du plâtre (sulfate de calcium hémihydraté) en sulfate de calcium dihydraté provoquant la solidification de la composition. L'utilisation de plâtre comme agent de solidification est avantageuse car elle permet de remplacer l'opération de compactage, habituellement nécessaire pour obtenir une composition sous forme solide à partir de produits pulvérulents, par une opération de moulage, dont la mise en oeuvre est plus simple, moins coûteuse et permet d'obtenir des formes plus diverses. US-A-5 049 376 et US-A 4 724 138 décrivent, par exemple, la fabrication d'une composition cosmétique solide à l'aide de plâtre à partir d'un mélange pulvérulent ne contenant pas de corps gras.

Mais l'utilisation du plâtre pour obtenir une composition cosmétique solide pose un problème : il est difficile d'incorporer dans une telle composition des matières premières hydrophobes difficilement mouillables, dont la présence est cependant essentielle pour la qualité cosmétique des produits finis. En effet, par exemple, il est nécessaire d'introduire des corps gras, ou des composés ayant les propriétés des corps gras, telles que des poudres enrobées par un corps gras, pour apporter le confort à l'application et l'adhésion sur la peau de la composition cosmétique ; il est souvent souhaitable également d'introduire des poudres hydrophobes, comme le talc, pour donner du glissant à la composition. En présence de ces composés hydrophobes, le mouillage de la poudre se fait mal, ce qui entraîne une certaine hétérogénéité de la composition solidifiée. Après séchage, le produit contient des grains ou des agglomérats qui sont gênants lors de l'application, ou encore, le produit contient des lacunes dues à des inclusions d'air lors du mélange de la composition avec l'eau, ce qui peut rendre cassant le produit moulé et nuire à son aspect.

Pour éviter cet inconvénient, on a proposé dans WO-86 00 798 d'introduire les corps gras sous forme d'émulsion dans l'eau. Ce procédé nécessite donc une étape supplémentaire de mise en émulsion des corps gras. De plus, la quantité d'agents tensioactifs nécessaires devient très élevée, dès que l'on utilise des charges pulvérulentes hydrophobes, puisqu'il faut introduire à la fois des agents tensioactifs pour mettre en émulsion les corps gras et pour mouiller les poudres hydrophobes. En outre, le produit solide obtenu n'est pas satisfaisant, car il est mou et cassant. On a également proposé de disperser les corps gras dans un solvant (voir EP-A-0 095 889): cependant, la présence d'un solvant perturbe la prise du plâtre ; de plus, le problème du mouillage lors du mélange avec la phase aqueuse n'est pas résolu.

Selon la présente invention, on a trouvé qu'en mélangeant avec une phase aqueuse un mélange pratiquement pulvérulent contenant le plâtre, des composés hydrophobes et une proportion suffisante de composés pulvérulents hydrophiles, le mouillage du mélange pulvérulent est satisfaisant, sans qu'il soit nécessaire d'ajouter des quantités importantes d'agent tensioactif; en outre, que la composition solide obtenue ne présente ni lacunes, ni agglomérats. De plus, ce procédé a l'avantage de permettre l'incorporation, en proportion élevée dans les compositions, de charges "diffusantes" telles que des billes de silice et de silicone ou des microsphères expansées en matériau thermoplastique ayant une masse spécifique de 15 à 200 kg/m³ comme celles en terpolymère acrylonitrile/chlorure de vinylidène/méthacrylate de méthyle commercialisées par la société "CASCO-NOBEL" sous la dénomination "EXPANCEL 551 DE".

Pour déterminer si, selon l'invention, une matière pulvérulente est "hydrophobe" ou "hydrophile", on effectue le test ci-après défini. On remplit avec 20 ml d'eau un tube à essai d'un diamètre de 20 mm. On verse 2 grammes de poudre dans le tube sans agiter et on observe le comportement de la poudre pendant 5 minutes au maximum. Si la poudre reste parfaitement en surface, elle est considérée comme "hydrophobe". Dans le cas contraire, elle est considérée comme "hydrophile".

Selon l'invention, on peut, soit mélanger l'ensemble des différents composés constituant le mélange pulvérulent, soit effectuer un prémélange des différents composés hydrophobes et un prémélange des différents composés hydrophiles. Quel que soit le mode de préparation utilisé, on désignera dans la suite de la description l'ensemble des composés hydrophobes par le terme "fraction hydrophobe" et l'ensemble des composés hydrophiles par le terme "fraction hydrophile".

La présente invention a donc pour objet un procédé de fabrication d'une composition cosmétique solide à l'aide de plâtre, caractérisé par le fait que :
- on prépare un mélange pulvérulent (P) constitué par :
   1) du sulfate de calcium hémihydraté (CaSO₄, 1/2 H₂O) sous forme de poudre,
   2) une fraction hydrophobe (FO) comprenant au moins un corps gras dans le groupe formé par les huiles minérales, animales, végétales, les esters d'acide carboxylique avec un alcool gras en C₁₀-C₂₂ ou avec un alcool inférieur, les alcools gras, les huiles de silicone, les gommes de silicone, les cires de silicone ainsi que les huiles fluorées et leurs dérivés et/ou au moins une matière pulvérulente traitée par enrobage ou greffage chimique de façon à lui donner des caractéristiques hydrophobes,
   3) une fraction hydrophile (FI) sous forme de poudre,
   la proportion pondérale FI/FO étant comprise entre 0,08 et 7,5, de préférence entre 0,40 et 3,25 ;
- on prépare une phase aqueuse (PA) sous forme liquide ;
- on malaxe le mélange pulvérulent (P) et la phase aqueuse (PA) dans une proportion pondérale P/PA comprise entre 0,2 et 2, de préférence entre 0,5 et 1,5 de façon à obtenir un mélange coulable ;
- on verse le mélange coulable dans un moule ;
- on le laisse durcir par hydratation du sulfate de calcium hémihydraté en sulfate de calcium dihydraté (CaSO₄, 2H₂O) et on démoule.

Le sulfate de calcium hémihydraté utilisé selon l'invention peut être sous sa forme α et/ou sous sa forme β . Il peut être mélangé à au moins un agent de modification du temps de prise tel que des retardateurs comme le citrate de sodium et des accélérateurs comme le gypse et les sulfates de sodium. La quantité de sulfate de calcium hémihydraté représente, de façon convenable, 15 à 35 % en poids, de préférence, 20 à 30 % en poids du mélange pulvérulent (P).

La fraction hydrophobe (FO) contient obligatoirement au moins un corps gras et/ou au moins une poudre traitée par enrobage ou greffage chimique de façon à lui donner les propriétés cosmétiques d'un corps gras.

Les corps gras utilisables sont tous ceux généralement utilisés dans les compositions cosmétiques. Parmi ces corps gras, on peut citer les huiles d'origine minérale telles que l'huile de vaseline ; les huiles d'origine animale telles que la lanoline ; les huiles d'origine végétale telles que l'huile de jojoba ; les esters d'acide carboxylique et d'alcool gras en C₁₀-C₂₂ ou avec un alcool inférieur tels que le citrate de triisocétyle, le myristate d'isopropyle, le tridécyl néopentanoate ; les alcools gras tels que l'alcool oléylique, l'alcool isostéarylique, l'octyldodécanol ; les huiles, gommes ou cires de silicone telles que les alkyldiméthicones ; les huiles fluorées et leurs dérivés, notamment siliconés.

Selon l'invention, les corps gras peuvent contenir au moins un additif et/ou au moins un actif cosmétique liposoluble. Parmi ces additifs ou actifs, on peut citer des polymères tels que le copolymère hexadécène/vinylpyrrolidone, des filtres solaires, des parfums, des agents conservateurs, des antioxydants et des vitamines liposolubles.

Les poudres traitées par enrobage ou greffage chimique, de façon à leur donner les propriétés cosmétiques des corps gras, sont des produits pulvérulents de nature aussi bien hydrophobe qu'hydrophile qui ont été traités par des produits hydrophobes, parmi lesquels on peut citer, par exemple, les silicones, les lipoaminoacides, les savons métalliques, les dérivés fluorés, les huiles minérales, la lécithine, le triisostéaroyl titanate d'isopropyle, le polyéthylène et le collagène et ses dérivés.

La fraction hydrophobe (FO) peut, éventuellement, contenir des poudres hydrophobes par nature, c'est-à-dire sans qu'il soit nécessaire de les traiter, telles que :
- le talc qui est un silicate de magnésium hydraté,
- les poudres de polymères hydrophobes, telles que la poudre des polyamides de type nylon, par exemple celle commercialisée sous la dénomination "ORGASOL 2002 ED NAT COS" par la société "ATOCHEM", la poudre de polyéthylène, par exemple celle commercialisée sous la dénomination "COATHYLENE HA 1681" par la société "PLAST LABOR" ; les microsphères expansées en matériau thermoplastique, par exemple celle commercialisée sous la dénomination "EXPANCEL 551 DE" par la société "CASCO-NOBEL", les poudres polyfluorées notamment de polytétrafluoroéthylène, par exemple celle commercialisée sous la dénomination "MP 1400" par la société "DU PONT DE NEMOURS", les poudres de silicone, par exemple celle commercialisée sous la dénomination "TOSPEARL" par la société "TOSHIBA", les poudres de copolymère acrylique, telles que celle commercialisée sous la dénomination "POLYTRAP Q5 6603" par la société "DOW CHEMICAL", ou encore les poudres de polystyrène telles que celle commercialisée sous la dénomination "POLYSPHERE 3 000 SP" par la société "PRESPERSE",
- les lipoaminoacides, par exemple la lauroyl lysine,
- le nitrure de bore, et
- les savons métalliques d'acides carboxyliques en C₈-C₂₂, plus particulièrement en C₁₂-C₁₈, par exemple les stéarates de zinc et de magnésium, le laurate de zinc ou le myristate de magnésium.

Dans la fraction hydrophobe (FO), le(s) corps gras et/ou la (les) matière(s) pulvérulente(s) traitée(s) représente(nt) au moins 1 % en poids. La fraction hydrophobe (FO) représente 10 à 60 % en poids, de préférence, 20 à 45 % en poids du mélange pulvérulent (P).

La fraction hydrophile (FI) contient avantageusement au moins une charge non colorée hydrophile non traitée et/ou au moins une charge non colorée traitée hydrophile et/ou au moins un pigment.

Parmi les charges non colorées hydrophiles, on peut citer :
- les micas, qui sont des silicates d'aluminium et de potassium de compositions variées, d'origine naturelle, tels que la muscovite, la phlogopite, la lépidolite, la biotite et la séricite, ou d'origine synthétique,
- l'oxychlorure de bismuth,
- les silices, qui peuvent être sous forme de plaquettes ou de sphères telles que la silice commercialisée sous la dénomination "SILICA BEADS SB 700" par la société "MIYOSHI",
- les poudres de polymères hydrophiles, qui sont d'origine synthétique comme les polyacrylates, par exemple celui commercialisé sous la dénomination "MICROPEARL M 100" par la société "MATSUMOTO", les polyamides acryliques tels que ceux commercialisés par la société "ORIS", les polyuréthannes tels que celui commercialisé sous la dénomination "PLASTIC POWDER D 800" par la société "TOSHIKI" ou qui sont d'origine naturelle, comme les dérivés de cellulose ou d'amidon, par exemple les microsphères poreuses de cellulose,
- le kaolin, qui est un silicate d'aluminium hydraté,
- l'hydroxyapatite,
- les oxydes de zinc ou de titane, pour leur couvrance notamment, ces produits pouvant être utilisés à l'état nanopigmentaire pour leur effet filtrant,
- le carbonate de calcium, et
- les carbonate et hydrocarbonate de magnésium, qui facilitent la fixation des parfums.

Les charges traitées hydrophiles sont des matières pulvérulentes traitées par enrobage ou greffage chimique, pour rendre leur surface hydrophile, à l'aide de matières, telles que le chitosane, le dioxyde de titane, la silice ou des polymères hydrophiles, notamment des polyesters sulfoniques ou des polyammonium quaternaire.

Les pigments peuvent être tout pigment coloré hydrophile utilisable en cosmétique. Ces pigments peuvent être des pigments minéraux, organiques ou des pigments nacrés enrobés ou non. Parmi les pigments minéraux, on peut citer, à titre d'exemple :
- les oxydes de fer noir, jaune, rouge et brun, codifiés dans le Color Index sous les références CI 77499, CI 77492 et CI 77491;
- le violet de manganèse (CI 77742);
- le bleu d'outremer (CI 77007);
- le violet d'outremer (CI 77007);
- l'oxyde de chrome (CI 77288);
- l'oxyde de chrome hydraté (CI 77289); et
- le bleu ferrique (CI 77510).
Parmi les pigments organiques, on peut citer, en particulier, les pigments:
- D & C red n° 3 (CI 45430 : 1)
- D & C red n° 6 (CI 15850 : 2)
- D & C red n° 7 (CI 15850 : 1)
- D & C red n° 9 (CI 15585 : 1)
- D & C red n° 13 (CI 15630 : 3)
- D & C red n° 19 (CI 45170)
- D & C red n° 21 (CI 45380 : 2)
- D & C red n° 27 (CI 45410 : 1)
- D & C red n° 30 (CI 73360)
- D & C red n° 36 (CI 12085)
- le noir de carbone (CI 77266) et les laques à base de carmin de cochenille (CI 75470).
Les pigments nacrés peuvent être choisis, notamment, parmi les pigments nacrés blancs, tels que le mica recouvert d'oxyde de titane ou d'oxychlorure de bismuth. On peut utiliser également des pigments nacrés colorés, tel que le mica titane coloré avec des oxydes de fer, le mica titane coloré avec du bleu ferrique ou de l'oxyde de chrome, le mica titane coloré avec un pigment organique du type précité, ainsi que des pigments nacrés à base d'oxychlorure de bismuth.

La fraction hydrophile (FI) représente généralement de 5 à 75 % en poids du mélange pulvérulent (P), de préférence de 25 à 60 % en poids.

Le mélange pulvérulent (P) peut éventuellement contenir au moins un agent tensioactif, qui pourra aider au mouillage et à la dispersion du mélange pulvérulent. L'agent tensioactif peut être nonionique comme les esters de sorbitane oxyéthylénés, cationique comme les sels d'ammonium quaternaire, ou amphotère comme les dérivés de bétaïne. La quantité d'agent tensioactif introduite ne dépasse pas 5 % en poids du mélange pulvérulent (P).

La phase aqueuse (PA) contient obligatoirement de l'eau en quantité suffisante pour hydrater le plâtre en sulfate de calcium dihydraté. Elle contient éventuellement au moins un additif soluble ou dispersible dans l'eau. Ces additifs peuvent être :
- des actifs cosmétiques solubles dans l'eau, tels que la glycérine, le polyéthylèneglycol, des filtres solaires ou des adjuvants solubles dans l'eau, tels que les conservateurs, les antioxydants ;
- des polymères hydrosolubles ou hydrodispersibles, tels que les polyammoniums quaternaires, les polyacrylates, les dérivés de vinylpyrrolidone, le chitosane ou les polyholosides, ces polymères pouvant être introduits sous forme de latex ;
- des cires hydrosolubles ou hydrodispersibles, telles que le silicone polyéther ou un polyéthylène dispersible ;
- des agents tensioactifs solubles ou dispersibles dans l'eau, qui peuvent être non-ioniques, cationiques ou amphotères, par exemple les diméthicone copolyols et les éthers d'alcools gras oxyéthylénés.

La présente invention a également pour objet la composition cosmétique solide contenant du plâtre obtenu par le procédé décrit ci-dessus.

Cette composition contient de 15 à 35 % en poids de sulfate de calcium dihydraté (CaSO₄,2H₂O), de préférence, de 20 à 30 % en poids; 10 à 60 % en poids, de préférence, 20 à 45 % en poids de fraction hydrophobe (FO) telle que décrite ci-dessus ; 5 à 75 % en poids, de préférence, 20 à 65 % en poids, de fraction hydrophile (FI) telle que décrite ci-dessus, le(s) corps gras et/ou la (ou les) matière(s) pulvérulente(s) traitée(s) par enrobage ou greffage chimique pour devenir hydrophobe(s) représentant au moins 0,1 % en poids de la composition.

Cette composition contient éventuellement au plus 10 % en poids d'agent tensioactif et/ou au plus 10 % en poids d'additifs cosmétiques préalablement dispersés ou solubilisés dans l'eau et/ou au plus 10 % en poids de modificateur du temps de prise.

Les exemples donnés ci-après, à titre illustratif et non limitatif, permettront de mieux comprendre l'invention.

### EXEMPLE 1: Fard à joues

On prépare un mélange pulvérulent (P) ayant la formulation suivante (en grammes) :

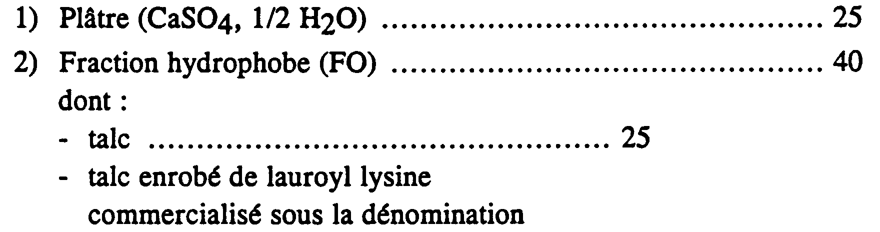

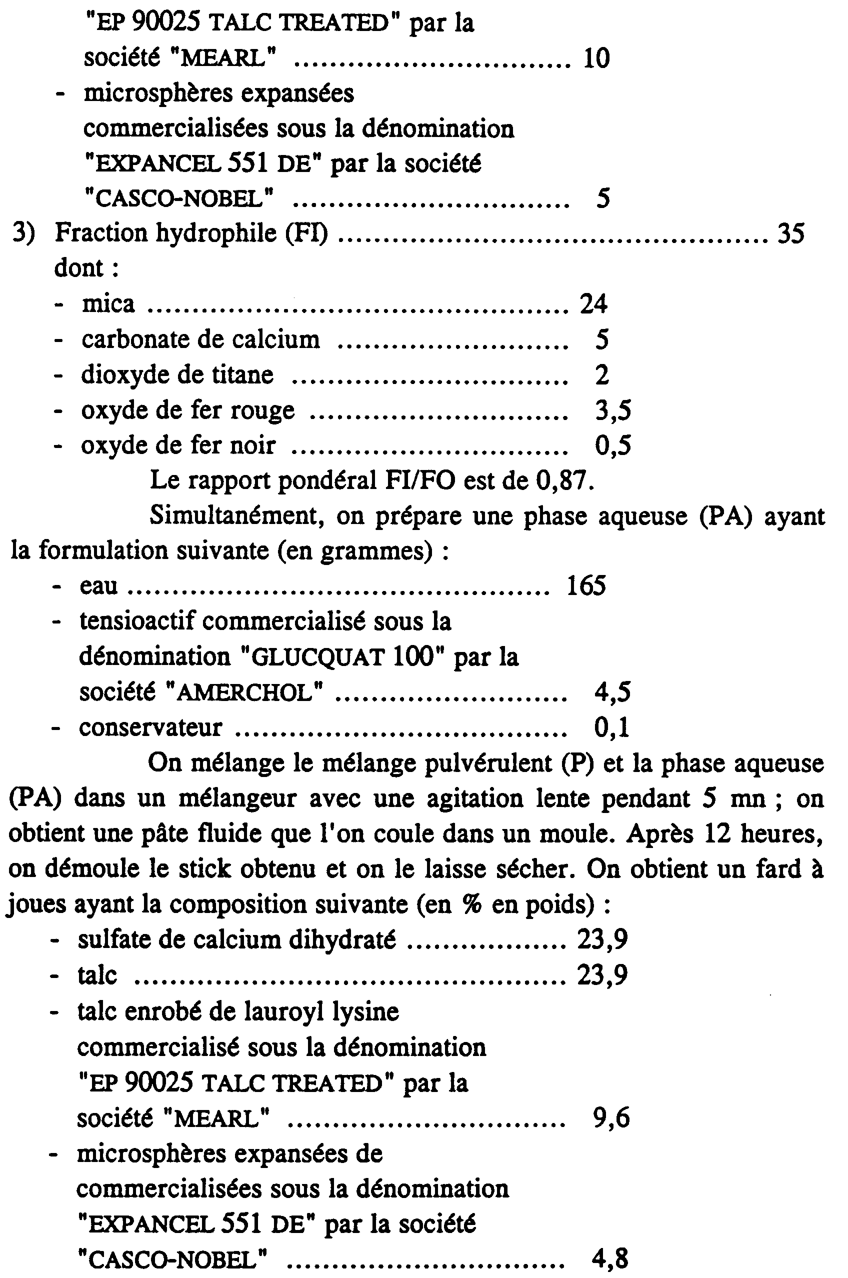

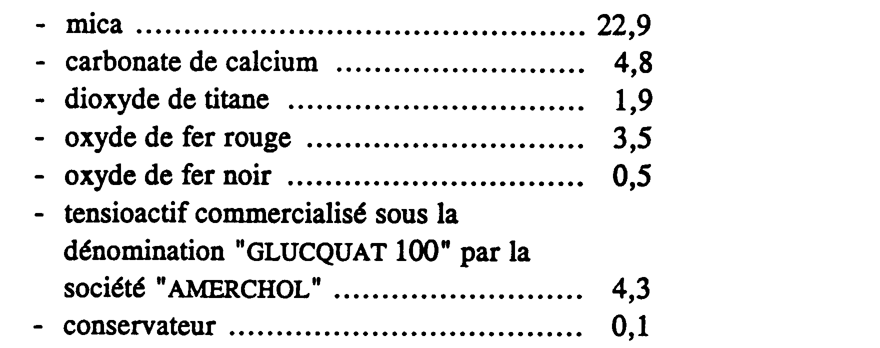

Le fard à joues obtenu ne contient ni aggrégat ni lacune ; il est dur mais délitable et peut être facilement appliqué.

### EXEMPLE 2 : Poudre pour le visage

On prépare, comme dans l'exemple 1, une pâte ayant la formulation donnée ci-après en grammes, qui donne, après moulage et séchage, un produit fini sec ayant la composition indiquée ci-après (en % en poids) :

| CONSTITUANTS | Pâte (en grammes) | Produit fini sec (en % en poids) |
|---|---|---|
| Mélange pulvérulent (P) | | |
| 1) Plâtre (CaSO₄, 1/2 H₂O) | 30 | 28,65* |
| 2) Fraction hydrophobe (FO) | | |
| - talc | 39,5 | 37,7 |
| - mica enrobé de silicone par le traitement SI commercialisé par la société "MIYOSHI" | 10 | 9,6 |
| - poudre de nylon commercialisée sous la dénomination "ORGASOL 2002 ED NAT COS" par la société "ATOCHEM" | 10 | 9,6 |
| 3) Fraction hydrophile (FI) | | |
| - oxychlorure de bismuth | 5 | 4,8 |
| - kaolin | 5 | 4,8 |
| - mélange d'oxydes de fer | 0,5 | 0,45 |

| Phase aqueuse (PA) | | |
|---|---|---|
| - eau | 125 | - |
| - tensioactif commercialisé sous la dénomination "BRIJ 99" par la société "ICI" | 4,5 | 4,3 |
| - gomme arabique | 0,1 | 0,1 |

| | | |
|---|---|---|
| * sous forme de (CaSO₄, 2H₂O) | | |

Le produit obtenu se présente, après moulage et séchage, sous forme d'un pain bombé, utilisable avec une houppette ; il ne contient ni agglomérat, ni lacune. La pâte peut aussi être moulée sous forme de crayon utilisable comme anti-cernes.

### EXEMPLE 3 : Poudre pour le visage

On prépare, comme dans l'exemple 1, une pâte ayant la formulation donnée ci-après en grammes, qui donne, après moulage et séchage, un produit fini sec ayant la composition indiquée ci-après (en % en poids) :

| CONSTITUANTS | Pâte (en grammes) | Produit fini sec (en % en poids) |
|---|---|---|
| Mélange pulvérulent (P) | | |
| 1) Plâtre (CaSO₄, 1/2 H₂O) | | |
| avec accélérateur (gypse) | 28 | 26,8 * |
| 2) Fraction hydrophobe (FO) | | |
| - talc | 21,6 | 20,6 |
| - nitrure de bore | 5 | 4,8 |
| - tensioactif commercialisé sous la dénomination "TWEEN 20" par la société "ICI" | 1 | 1 |
| - parfum | 0,2 | 0,2 |
| - polydiméthylsiloxane commercialisé sous la dénomination "ABIL 10" par la société "GOLDSCHMIDT" | 5 | 4,8 |
| 3) Fraction hydrophile (FI) | | |
| - mica | 22 | 21 |
| - poudre de polyméthylméthacrylate commercialisée sous la dénomination "MICROPEARL M 305" par la société "MATSUMOTO" | 17 | 16,3 |
| - mélange d'oxydes de fer | 0,2 | 0,2 |

| Phase aqueuse (PA) | | |
|---|---|---|
| - eau | 125 | - |
| - polyéthylèneglycol comportant 8 unités oxyde d'éthylène | 4,5 | 4,3 |

| | | |
|---|---|---|
| * sous forme de (CaSO₄, 2H₂O) | | |

La poudre obtenue se présente sous forme d'un pain rectangulaire utilisable avec une houppette ou un pinceau, ne présentant ni agglomérat, ni lacune.

### EXEMPLE 4 : Fard à paupières

On prépare, comme dans l'exemple 1, une pâte ayant la formulation donnée ci-après en grammes, qui donne, après moulage et séchage, un produit fini sec ayant la composition indiquée ci-après (en % en poids) :

| CONSTITUANTS | Pâte (en grammes) | Produit fini sec (en % en poids) |
|---|---|---|
| Mélange pulvérulent (P) | | |
| 1) Plâtre (CaSO₄, 1/2 H₂O) | 20 | 19,9 * |
| 2) Fraction hydrophobe (FO) | | |
| - talc | 23 | 22,9 |
| - huile de vaseline | 0,9 | 0,9 |
| - copolymère hexadécène/vinylpyrrolidone commercialisé sous la dénomination "GANEX "V216" par la société "ISP" | 0,1 | 0,1 |
| 3) Fraction hydrophile (FI) | | |
| - microbilles de silice commercialisées sous la dénomination "SILICA BEADS SB 150" par la société "MIYOSHI" | 20 | 19,9 |
| - oxychlorure de bismuth | 10 | 9,9 |
| - mica-titane | 20 | 19,9 |
| - bleu ferrique | 6 | 5,9 |

| Phase aqueuse (PA) | | |
|---|---|---|
| - eau | 124 | - |
| - conservateurs | 0,2 | 0,2 |
| - polyvinylpyrrolidone | 0,4 | 0,4 |

| | | |
|---|---|---|
| * sous forme de (CaSO₄, 2H₂O) | | |

Le fard obtenu est sous forme d'une pastille bombée utilisable au doigt ou avec un applicateur, et qui ne présente ni agglomérat, ni lacune.

## Revendications

1. Procédé de fabrication d'une composition cosmétique solide à l'aide de plâtre, caractérisé par le fait que :
- on prépare un mélange pulvérulent (P) constitué par :
1) du sulfate de calcium hémihydraté (CaSO₄, 1/2 H₂O) sous forme de poudre,
2) une fraction hydrophobe (FO) comprenant au moins un corps gras choisi dans le groupe formé par les huiles minérales, animales, végétales, les esters d'acide carboxylique avec un alcool gras en C₁₀-C₂₂ ou avec un alcool inférieur, les alcools gras, les huiles de silicone, les gommes de silicone, les cires de silicone ainsi que les huiles fluorées et leurs dérivés et/ou au moins une matière pulvérulente traitée par enrobage ou greffage chimique de façon à lui donner des caractéristiques hydrophobes,
3) une fraction hydrophile (FI) sous forme de poudre,
la proportion pondérale FI/FO étant comprise entre 0,08 et 7,5 ;
- on prépare une phase aqueuse (PA) sous forme liquide ;
- on malaxe le mélange pulvérulent (P) et la phase aqueuse (PA) dans une proportion pondérale P/PA comprise entre 0,2 et 2, de façon à obtenir un mélange coulable ;
- on verse le mélange coulable dans un moule ;
- on le laisse durcir par hydratation du sulfate de calcium hémihydraté en sulfate de calcium dihydraté (CaSO₄, 2H₂O) et on démoule.

2. Procédé selon la revendication 1, caractérisé par le fait qu'on malaxe le mélange pulvérulent (P) et la phase aqueuse (PA) dans une proportion pondérale comprise entre 0,5 et 1,5.

3. Procédé selon l'une des revendications 1 ou 2, caractérisé par le fait qu'on amène par la phase aqueuse (PA) au mélange (P) une quantité d'eau au moins suffisante pour hydrater tout le sulfate de calcium hémihydraté en sulfate de calcium dihydraté.

4. Procédé selon l'une des revendications 1 à 3, caractérisé par le fait qu'on choisit une proportion pondérale de la fraction hydrophile (FI) par rapport à la fraction hydrophobe (FO) comprise entre 0,40 et 3,25.

5. Procédé selon l'une des revendications 1 à 4, caractérisé par le fait que l'on mélange le sulfate de calcium hémihydraté à au moins un agent de modification du temps de prise.

6. Procédé selon l'une des revendications 1 à 5, caractérisé par le fait que le sulfate de calcium hémihydraté représente de 15 à 35 % en poids du mélange pulvérulent (P).

7. Procédé selon la revendication 1, caractérisé par le fait que le corps gras contient au moins un additif et/ou au moins un actif liposoluble.

8. Procédé selon l'une des revendications 1 à 7, caractérisé par le fait que l'on choisit les matières pulvérulentes traitées par enrobage ou greffage chimique dans le groupe formé par les poudres traitées par des silicones, des lipoaminoacides, des savons métalliques, des dérivés fluorés, des huiles minérales, de la lécithine, du triisostéaroyl titanate d'isopropyle, du polyéthylène et du collagène et ses dérivés.

9. Procédé selon l'une des revendications 1 à 8, caractérisé par le fait que la fraction hydrophobe (FO) du mélange pulvérulent (P) contient au moins une matière pulvérulente hydrophobe, non traitée par enrobage ou greffage, choisie dans le groupe formé par le talc, les poudres de polymères hydrophobes, les lipoaminoacides, le nitrure de bore et les savons métalliques d'acides carboxyliques en C₈-C₂₂.

10. Procédé selon la revendication 9, caractérisé par le fait que les poudres de polymères hydrophobes sont choisies dans le groupe formé par les poudres de polyamide, de polyéthylène, les microsphères expansées en matériau thermoplastique, les poudres polyfluorées, les poudres de silicone, les poudres de copolymère acrylique et les poudres de polystyrène.

11. Procédé selon l'une des revendications 9 ou 10, caractérisé par le fait que le polymère hydrophobe est un terpolymère acrylonitrile/chlorure de vinylidène/méthacrylate de méthyle.

12. Procédé selon l'une des revendications 1 à 11, caractérisé par le fait que le(s) corps gras et/ou la (les) matière(s) pulvérulente(s) traitée(s) représente(nt) au moins 1 % en poids de la fraction hydrophobe (FO).

13. Procédé selon l'une des revendications 1 à 12, caractérisé par le fait que la fraction hydrophobe (FO) représente de 10 à 60 % en poids du mélange pulvérulent (P).

14. Procédé selon l'une des revendications 1 à 13, caractérisé par le fait que l'on choisit les constituants de la fraction hydrophile (FI) dans le groupe formé par des charges non colorées hydrophiles non traitées, des charges non colorées traitées hydrophiles et des pigments enrobés ou non.

15. Procédé selon la revendication 14, caractérisé par le fait que l'on choisit les charges non colorées hydrophiles dans le groupe formé par les micas, l'oxychlorure de bismuth, les silices, les poudres de polymères hydrophiles, le kaolin, l'hydroxyapatite, l'oxyde de zinc, l'oxyde de titane, le carbonate de calcium, le carbonate de magnésium et l'hydrocarbonate de magnésium.

16. Procédé selon la revendication 14, caractérisé par le fait que l'on choisit les charges non colorées traitées hydrophiles dans le groupe formé par les poudres traitées par le chitosane, le dioxyde de titane, la silice et les polymères hydrophiles.

17. Procédé selon la revendication 16, caractérisé par le fait que l'on choisit les pigments dans le groupe formé par les pigments minéraux, organiques et nacrés.

18. Procédé selon l'une des revendications 1 à 17, caractérisé par le fait que la fraction hydrophile (FI) représente de 5 à 75 % en poids du mélange pulvérulent (P), de préférence 25 à 60 %.

19. Procédé selon l'une des revendications 1 à 18, caractérisé par le fait que le mélange pulvérulent (P) contient au moins un agent tensioactif choisi dans le groupe formé par les agents tensioactifs non-ioniques, cationiques et amphotères.

20. Procédé selon l'une des revendications 1 à 19, caractérisé par le fait que la phase aqueuse (PA) contient au moins un additif soluble ou dispersible dans l'eau.

21. Procédé selon la revendication 20, caractérisé par le fait que l'on choisit l'additif dans le groupe formé par les actifs cosmétiques solubles dans l'eau, les polymères, les cires et les tensioactifs hydrosolubles ou hydrodispersibles.

22. Composition cosmétique solide susceptible d'être obtenue par le procédé de l'une des revendications 1 à 21, caractérisée par le fait qu'elle contient 15 à 35 % en poids de sulfate de calcium dihydraté (CaSO₄, 2H₂O), 10 à 60 % en poids de constituants de la fraction hydophobe (FO), 5 à 75 % en poids de constituants de la fraction hydrophile (FI), le(s) corps gras et/ou la (ou les) matière(s) pulvérulente(s) traitée(s) par enrobage ou greffage chimique pour devenir hydrophobe(s) représentant au moins 0,1 % en poids de la composition.

23. Composition selon la revendication 22, caractérisée par le fait qu'elle contient 20 à 30 % en poids de sulfate de calcium dihydraté (CaSO₄, 2H₂O), 20 à 45 % en poids de constituants de la fraction hydrophobe (FO), et 20 à 65 % en poids de constituants de la fraction hydrophile (FI).

24. Composition selon l'une des revendications 22 ou 23, caractérisée par le fait qu'elle contient au plus 10 % en poids de tensioactifs et/ou au plus 10 % en poids d'additif(s) cosmétique(s) préalablement dispersé(s) ou solubilisé(s) dans l'eau et/ou au plus 10 % en poids de modificateur de temps de prise.

## Claims

1. Process for the manufacture of a solid cosmetic composition using plaster of Paris, characterized in that:
- a pulverulent mixture (P) consisting of:
1) calcium sulphate hemihydrate (CaSO₄.1/2 H₂O) in powder form
2) a hydrophobic fraction (OF) comprising at least one fatty substance in the group composed of mineral, animal and vegetable oils, esters of a carboxylic acid with a C₁₀-C₂₂ fatty alcohol or with a lower alcohol, fatty alcohols, silicone oils, silicone gums, silicone waxes also fluorinated oils and their derivatives, and/or at least one pulverulent substance treated by coating or chemical grafting so as to give it hydrophobic properties,
3) a hydrophilic fraction (IF) in powder form, is prepared, the weight proportion IF/OF being between 0.08 and 7.5;
- an aqueous phase (AP) in liquid form is prepared;
- the pulverulent mixture (P) and the aqueous phase (AP) are kneaded in a weight proportion P/AP of between 0.2 and 2, so as to obtain a pourable mixture;
- the pourable mixture is run into a mould;
- it is left to harden by hydration of the calcium sulphate hemihydrate to calcium sulphate dihydrate (CaSO₄2H₂O), and the product is removed from the mould.

2. Process according to Claim 1, characterized in that the pulverulent mixture (P) and the aqueous phase (AP) are kneaded in a weight proportion of between 0.5 and 1.5.

3. Process according to either of Claims 1 and 2, characterized in that an amount of water which is at least sufficient to hydrate all the calcium sulphate hemihydrate to calcium sulphate dihydrate is supplied by the aqueous phase (AP) to the mixture (P).

4. Process according to one of Claims 1 to 3, characterized in that a weight proportion of the hydrophilic fraction (IF) relative to the hydrophobic fraction (OF) of between 0.40 and 3.25 is chosen.

5. Process according to one of Claims 1 to 4, characterized in that the calcium sulphate hemihydrate is mixed with at least one setting time-modifying agent.

6. Process according to one of Claims 1 to 5, characterized in that the calcium sulphate hemihydrate represents from 15 to 35% by weight of the pulverulent mixture (P).

7. Process according to Claim 1, characterized in that the fatty substance contains at least one additive and/or at least one active agent which is fat-soluble.

8. Process according to one of Claims 1 to 7, characterized in that the pulverulent substances treated by coating or chemical grafting are chosen from the group composed of powders treated with silicones, with lipoamino acids, with metallic soaps, with fluorinated derivatives, with mineral oils, with lecithin, with isopropyl triisostearoyltitanate, with polyethylene and with collagen and its derivatives.

9. Process according to one of Claims 1 to 8, characterized in that the hydrophobic fraction (OF) of the pulverulent mixture (P) contains at least one hydrophobic pulverulent substance not treated by coating or grafting, chosen from the group composed of talc, powders of hydrophobic polymers, lipoamino acids, boron nitride and metallic soaps of C₈-C₂₂ carboxylic acids.

10. Process according to Claim 9, characterized in that the powders of hydrophobic polymers are chosen from the group composed of polyamide and polyethylene powders, expanded microspheres made of thermoplastic material, polyfluorinated powders, silicone powders, acrylic copolymer powders and polystyrene powders.

11. Process according to either of Claims 9 and 10, characterized in that the hydrophobic polymer is an acrylonitrile/vinylidene chloride/methyl methaaylateterpolymer.

12. Process according to one of Claims 1 to 11, characterized in that the fatty substance(s) and/or the treated pulverulent substance(s) represent(s) at least 1% by weight of the hydrophobic fraction (OF).

13. Process according to one of Claims 1 to 12, characterized in that the hydrophobic fraction (OF) represents from 10 to 60% by weight of the pulverulent mixture (P).

14. Process according to one of Claims 1 to 13, characterized in that the constituents of the hydrophilic fraction (IF) are chosen from the group composed of untreated hydrophilic uncoloured fillers, hydrophilic treated uncoloured fillers and pigments, coated or otherwise.

15. Process according to Claim 14, characterized in that the hydrophilic uncoloured fillers are chosen from the group composed of micas, bismuth oxychloride, silicas, powders of hydrophilic polymers, kaolin, hydroxyapatite, zinc oxide, titanium oxide, calcium carbonate, magnesium carbonate and basic magnesium carbonate.

16. Process according to Claim 14, characterized in that the hydrophilic treated uncoloured fillers are chosen from the group composed of powders treated with chitosan, titanium dioxide, silica and hydrophilic polymers.

17. Process according to Claim 14, characterized in that the pigments are chosen from the group composed of inorganic, organic and nacreous pigments.

18. Process according to one of Claims 1 to 17, characterized in that the hydrophilic fraction (IF) represents from 5 to 75% by weight of the pulverulent mixture (P), and preferably 25 to 60 %.

19. Process according to one of Claims 1 to 18, characterized in that the pulverulent mixture (P) contains at least one surfactant chosen from the group composed of nonionic, cationic and amphoteric surfactants.

20. Process according to one of Claims 1 to 19, characterized in that the aqueous phase (AP) contains at least one water-soluble or -dispersible additive.

21. Process according to Claim 20. characterized in that the additive is chosen from the group composed of water-soluble cosmetic active agents, polymers, waxes and water-soluble or water-dispersible surfactants.

22. Solid cosmetic composition capable of being obtained by the process of one of Claims 1 to 21, characterized in that it contains 15 to 35% by weight of calcium sulphate dihydrate (CaSO₄.2H₂O), 10 to 60% by weight of constituents of the hydrophobic fraction (OF) and 5 to 75% by weight of constituents of the hydrophilic fraction (IF), the fatty substance(s) and/or the pulverulent substance(s) treated by coating and/or chemical grafting so as to become hydrophobic representing at least 0.1% by weight of the composition.

23. Composition according to Claim 22, characterized in that it contains 20 to 30% by weight of calcium sulphate dihydrate (CaSO₄.2H₂O) 20 to 45% by weight of constituents of the hydrophobic fraction (OF) and 20 to 65% of weight of constituents of the hydrophilic fraction (IF).

24. Composition according to either of Claims 22 and 23, characterized in that it contains at most 10% by weight of surfactants and/or at most 10% by weight of cosmetic additive(s) previously dispersed or solubilized in water, and/or at most 10% by weight of setting time-modifying agent.

## Patentansprüche

1. Verfahren zur Herstellung einer festen kosmetischen Zusammensetzung mit Hilfe von Gips, dadurch gekennzeichnet, daß man:
- ein pulverförmiges Gemisch (P) aus folgenden Bestandteilen herstellt:
1) Calciumsulfat-Hemihydrat (CaSO₄, 1/2 H₂O) in Pulverform,
2) einer hydrophoben Fraktion (FO), die wenigstens einen unter Mineralölen, Tierölen, Pflanzenölen, Estern einer Carbonsäure mit einem C₁₀-C₂₂-Fettalkohol oder mit einem niederen Alkohol, Fettalkoholen, Silikonölen, Silikongummis, Silikonwachsen sowie fluorierten Ölen und deren Derivaten ausgewählten Fettkörper und/oder wenigstens einen pulverförmigen Stoff umfaßt, der durch chemisches Aufpropfen oder Umhüllen so behandelt worden ist, daß ihm hydrophobe Eigenschaften verliehen werden,
3) einer hydrophilen Fraktion (FI) in Pulverform,
wobei das Gewichtsverhältnis FI/FO 0,08 bis 7,5 beträgt;
- eine wäßrige Phase (PA) in flüssiger Form herstellt;
- das pulverförmige Gemisch (P) und die wäßrige Phase (PA) in einem Gewichtsverhältnis P/PA von 0,2 bis 2 so miteinander verknetet, daß man ein gießfähiges Gemisch erhält;
- das gießfähige Gemisch in eine Form gibt;
- dieses durch Hydratation des Calciumsulfat-Hemihydrates zum Calciumsulfat-Dihydrat (CaSO₄, 2H₂O) fest werden läßt und aus der Form nimmt.

2. Verfahren nach Anspruch 1, dadurch gekennzeichnet, daß man das pulverförmige Gemisch (P) und die wäßrige Phase (PA) in einem Gewichtsverhältnis von 0,5 bis 1,5 miteinander verknetet.

3. Verfahren nach einem der Ansprüche 1 oder 2, dadurch gekennzeichnet, daß man mit der wäßrigen Phase (PA) soviel Wasser in das Gemisch (P) miteinbringt, daß das gesamte Calciumsulfat-Hemihydrat zum Calciumsulfat-Dihydrat hydratisiert wird.

4. Verfahren nach einem der Ansprüche 1 bis 3, dadurch gekennzeichnet, daß man ein Gewichtsverhältnis von hydrophiler Fraktion (FI) zu hydrophober Fraktion (FO) von 0,40 bis 3,25 wählt.

5. Verfahren nach einem der Ansprüche 1 bis 4, dadurch gekennzeichnet, daß man das Calciumsulfat-Hemihydrat mit wenigstens einem Mittel zur Modifizierung der Abbindezeit vermischt.

6. Verfahren nach einem der Ansprüche 1 bis 5, dadurch gekennzeichnet, daß das Calciumsulfat-Hemihydrat 15 bis 35 Gew.-% des pulverförmigen Gemisches (P) ausmacht.

7. Verfahren nach Anspruch 1, dadurch gekennzeichnet, daß der Fettkörper wenigstens einen fettlöslichen Wirkstoff und/oder wenigstens ein Additiv enthält.

8. Verfahren nach einem der Ansprüche 1 bis 7, dadurch gekennzeichnet, daß man die pulverförmigen, durch chemisches Aufpropfen oder Umhüllen behandelten Stoffe unter Pulvern auswählt, die mit Silikonen, Lipoaminosäuren, Metallseifen, fluorierten Derivaten, Mineralölen, Lecithin, Isopropyltriisostearoyltitanat, Polyethylen und Collagen und dessen Derivaten behandelt worden sind.

9. Verfahren nach einem der Ansprüche 1 bis 8, dadurch gekennzeichnet, daß die hydrophobe Fraktion (FO) des pulverförmigen Gemisches (P) wenigstens einen hydrophoben pulverförmigen Stoff enthält, der nicht durch Aufpropfen oder Umhüllen behandelt worden ist und unter Talk, Pulvern aus hydrophoben Polymeren, Lipoaminosäuren, Bornitrid und Metallseifen von C₈-C₂₂-Carbonsäuren ausgewählt ist.

10. Verfahren nach Anspruch 9, dadurch gekennzeichnet, daß die Pulver aus hydrophoben Polymeren unter Polyamidpulvern, Polyethylenpulvern, expandierten Mikrosphären aus thermoplastischem Material, polyfluorierten Pulvern, Silikonpulvern, Pulvern aus Acrylcopolymeren und Polystyrolpulvern ausgewählt sind.

11. Verfahren nach einem der Ansprüche 9 oder 10, dadurch gekennzeichnet, daß das hydrophobe Polymer ein Acrylnitril/Vinylidenchlorid/Methylmethacrylat-Terpolymer ist.

12. Verfahren nach einem der Ansprüche 1 bis 11, dadurch gekennzeichnet, daß der (die) Fettkörper und/oder der (die) behandelte(n) pulverförmige(n) Stoff(e) wenigstens 1 Gew.-% der hydrophoben Fraktion (FO) ausmacht (ausmachen).

13. Verfahren nach einem der Ansprüche 1 bis 12, dadurch gekennzeichnet, daß die hydrophobe Fraktion (FO) 10 bis 60 Gew.-% des pulverförmigen Gemisches (P) ausmacht.

14. Verfahren nach einem der Ansprüche 1 bis 13, dadurch gekennzeichnet, daß man die Bestandteile der hydrophilen Fraktion (FI) unter unbehandelten hydrophilen ungefärbten Füllstoffen, hydrophilen behandelten ungefärbten Füllstoffen und umhüllten oder nicht-umhüllten Pigmenten auswählt.

15. Verfahren nach Anspruch 14, dadurch gekennzeichnet, daß man die hydrophilen ungefärbten Füllstoffe unter Glimmern, Bismutoxidchlorid, Kieselerden, Pulvern aus hydrophilen Polymeren, Kaolin, Hydroxyapatit, Zinkoxid, Titanoxid, Calciumcarbonat, Magnesiumcarbonat und Magnesiumhydrogencarbonat auswählt.

16. Verfahren nach Anspruch 14, dadurch gekennzeichnet, daß man die hydrophilen behandelten ungefärbten Füllstoffe unter Pulvern auswählt, die mit Chitosan, Titandioxid, Kieselerde und hydrophilen Polymeren behandelt sind.

17. Verfahren nach Anspruch 16, dadurch gekennzeichnet, daß man die Pigmente unter mineralischen, organischen und perlmuttartigen Pigmenten auswählt.

18. Verfahren nach einem der Ansprüche 1 bis 17, dadurch gekennzeichnet, daß die hydrophile Fraktion (FI) 5 bis 75 Gew.-% und vorzugsweise 25 bis 60 Gew.-% des pulverförmigen Gemisches (P) ausmacht.

19. Verfahren nach einem der Ansprüche 1 bis 18, dadurch gekennzeichnet, daß das pulverförmige Gemisch (P) wenigstens ein oberflächenaktives Mittel enthält, das unter nichtionischen, kationischen und amphoteren oberflächenaktiven Mitteln ausgewählt ist.

20. Verfahren nach einem der Ansprüche 1 bis 19, dadurch gekennzeichnet, daß die wäßrige Phase (PA) wenigstens ein in Wasser lösliches oder dispergierbares Additiv enthält.

21. Verfahren nach Anspruch 20, dadurch gekennzeichnet, daß man das Additiv unter in Wasser löslichen kosmetischen Wirkstoffen, Polymeren, Wachsen und wasserlöslichen oder wasserdispergierbaren oberflächenaktiven Mitteln auswählt.

22. Feste kosmetische Zusammensetzung, erhältlich nach einem Verfahren der Ansprüche 1 bis 21, dadurch gekennzeichnet, daß sie 15 bis 35 Gew.-% Calciumsulfat-Dihydrat (CaSO₄, 2H₂O), 10 bis 60 Gew.-% an Bestandteilen der hydrophoben Fraktion (FO) und 5 bis 75 Gew.-% an Bestandteilen der hydrophilen Fraktion (FI) enthält, wobei der (die) Fettkörper und/oder der (oder die) pulverförmige(n) Stoff(e), der (die) durch chemisches Aufpropfen oder Umhüllen behandelt worden ist (sind), um hydrophob zu werden, wenigstens 0,1 Gew.-% der Zusammensetzung ausmachen.

23. Zusammensetzung nach Anspruch 22, dadurch gekennzeichnet, daß sie wenigstens 20 bis 30 Gew.-% Calciumsulfat-Dihydrat (CaSO₄, 2H₂O), 20 bis 45 Gew.-% an Bestandteilen der hydrophoben Fraktion (FO) und 20 bis 65 Gew.-% an Bestandteilen der hydrophilen Fraktion (FI) enthält.

24. Zusammensetzung nach einem der Ansprüche 22 oder 23, dadurch gekennzeichnet, daß sie wenigstens 10 Gew.-% an oberflächenaktiven Mitteln und/oder wenigstens 10 Gew.-% an zuvor in Wasser dispergierten oder solubilisierten kosmetischen Additiven und/oder wenigstens 10 Gew.-% eines Mittel zur Modifizierung der Abbindezeit enthält.
